# EUROPÄISCHE PATENTSCHRIFT

(11) **EP 1 328 237 B1**
(45) Veröffentlichungstag und Bekanntmachung des Hinweises auf die Patenterteilung: **28.12.2005**
(21) Anmeldenummer: 01983544.6
(22) Anmeldetag: 11.10.2001
(51) Int. Cl.: A61K 6/10

(54) **VORRICHTUNG ZUR BESTIMMUNG DES ENDES DER VERARBEITUNGSZEIT VON HÄRTBAREN MASSEN**
DEVICE FOR DETERMINING THE END OF THE PROCESSING TIME OF HARDENABLE MATERIALS
DISPOSITIF POUR DETERMINER LA FIN DU TEMPS DE FACONNAGE DE MATIERES DURCISSABLES

(30) Priorität: 23.10.2000 DE 10052542
(43) Veröffentlichungstag der Anmeldung: 23.07.2003
(73) Patentinhaber: 3M ESPE AG, 82229 Seefeld (DE)
(72) Erfinder: BRANDHORST, Gerd, 86899 Landsberg (DE); HERTLEIN, Günter, 82110 Germering (DE); NIRSCHL, Hermann, 82229 Seefeld (DE); PEUKER, Marc, 86938 Schondorf (DE); WAGNER, Ingo, 82237 Wörthsee (DE); WANEK, Erich, 86916 Kaufering (DE)
(74) Vertreter: Brem, Roland
(86) Internationale Anmeldenummer: PCT/EP2001/011767
(87) Internationale Veröffentlichungsnummer: WO 2002/034208

(56) Entgegenhaltungen:
- WO-A-96/00560
- DE-A- 3 400 781
- DE-A- 19 741 674
- DE-C- 19 903 753
- DE-U- 29 906 343
- US-A- 5 063 255

## Beschreibung

Die Erfindung betrifft eine Vorrichtung und die Verwendung dieser Vorrichtung enthaltend eine Sensor sowie ein Verfahren zur Bestimmung des Endes der Verarbeitungszeit von härtbaren Dentalmassen, insbesondere von dentalen Abformmassen.

Zur Verarbeitung und Anwendung von Abformmassen im Dentalbereich werden üblicherweise in den Gebrauchsanweisungen entsprechende Zeiten angegeben, wie die Abformmasse zu handhaben ist und wann sie aus dem Mund des Patienten entnommen werden soll.

Die angegebenen Zeiten für Verarbeitung und Abbindeverhalten der Massen sind jedoch in der zahnärztlichen Praxis verschiedenen Störgrößen unterworfen, wie die aktuelle Mund- und Raumtemperatur, eingebrachte Mischenergie oder die Zeit, die zum Mischen aufgewandt wurde.

Denkbar ist es, in die polymerisierbaren Massen Indikatoren einzuarbeiten, die während der Polymerisation freigesetzt werden und den Reaktionsfortgang beispielsweise durch Änderung der Farbintensität anzeigen. Ein solcher Versuch ist in der WO-96/00560 A2 beschrieben.

Nachteilig daran ist, dass die beschriebenen Massen eine weitere Komponente enthalten, die sich negativ auf die gewünschten Eigenschaften auswirken kann. Außerdem liefert die sich über die Zeit verändernde Farbe kein eindeutig derinierbares Signal über den Abbindevorgang und erfordert zudem eine ständige optische Kontrolle.

Die DE 29 906 343 U1 versucht das Problem durch Bereitstellung eines Gerätes zum Ausgeben von Mehrkomponentenmassen zu lösen, das mit einer Zeituhr zur Angabe einer für die Verarbeitung relevanten Zeit ausgerüstet ist.

Das beschriebene Gerät hat allerdings den Nachteil, dass die gemischte Masse ebenfalls den genannten äußeren Einflüssen unterliegt und somit keinen eindeutiger Hinweis gegeben wird, wann der Abbindevorgang einsetzt.

Vorrichtungen, mit denen Veränderungen rheologischer Eigenschaften erfasst werden können, sind aus der DE 19 741 674 A1 oder der DE 19 903 753 A1 bekannt. Diese Vorrichtungen eignen sich allerdings schon allein dimensionsbedingt nicht für eine Anwendung im Dentalbereich.

Folglich ist es eine Aufgabe der vorliegenden Erfindung, eine Vorrichtung zur Verfügung zu stellen, die dem Anwender das Ende der Verarbeitungszeit von härtbaren bzw, erhärtenden Massen anzeigt.

Diese Aufgabe wird durch die Verwendung eines Sensors und durch Bereitstellung einer geeigneten Vorrichtung gelöst, wie sie in den Ansprüchen beschrieben sind.

Mit den Begriffen "umfassen" oder "enthalten" wird eine nicht abschließende Aufzählung von Merkmalen eingeleitet. Der Umstand, dass in den Ansprüchen das Wort "ein" vor Nennung eines Merkmals verwendet wird, schließt nicht aus, dass die genannten Merkmale mehrmals vorhanden sein können, im Sinne von "mindestens ein".

Unter dem Ausdruck Ende der Verarbeitungszeit im Sinne der Erfindung ist die Zeit zu verstehen, nach deren Ablauf die härtbare "Dentalmasse bestimmungsgemäß im wesentlichen vollständig ausgehärtet ist und im wesentlichen keine Änderungen rheologischer Eigenschaften mehr beobachtbar und/oder initiierbar sind.

Der Ausdruck härtbare "Dentalmassen umfasst alle Massen, die in Folge einer Polymerisationsreaktion, beispielsweise einer radikalisch, kationisch oder anionisch verlaufenden Additions- und/oder Kondensationsreaktion, und/oder Zementreaktion von einem viskosen, fließfähigen, ggf. plastisch verformbaren Zustand in einen dauerhaft verformten festen Zustand übergehen können.

Der Ausdruck härtbare Dentalmassen umfasst, insbesondere dentale Abformmassen auf der Basis von Polyethern, A- und C-Silikonen, Alginate und/oder Polyethersilikonen.

Bei Abformmassen liegt die Masse nach dem Härten üblicherweise in einer Negativform der abgeformten Oberfläche vor.

Massen, deren Abbindevorgang mit der erfindungsgemäßen Vorrichtung vorzugsweise erfassbar ist, weisen vor Beginn des Abbindevorgangs üblicherweise folgende Eigenschaften auf: Es handelt sich um pastöse, hochviskose Substanzen, die nach Initiierung einer Härtungsreaktion über einen Zeitraum im Bereich von 0,1 bis 60, vorzugsweise 1 bis 8 min erstarren.

Solche Massen haben beispielsweise vor Beginn des Abbindevorgangs eine Viskosität bestimmt durch Konsistenzprüfung nach DIN 4823 Klasse 0 bis 3, die mit einem Durchmesser von kleiner 80 mm gemessen werden. Als Materialien seien beispielhaft genannt Silikone, Polyether, Epoxide und Polyurethane.

Die Shore-Härte A der Masse, gemessen nach DIN 53505 15 min nach Ende der Verarbeitungszeit, liegt üblicherweise im Bereich von 20 bis 110, vorzugsweise im Bereich von 30 und 80.

Unter Eigenschaften, insbesondere rheologische Eigenschaften sind alle Eigenschaften zu verstehen, deren Veränderung über ein physikalisch und/oder chemisches Messverfahren erfasst werden kann. Hierunter fallen insbesondere die Eigenschaften Dielektrizitätskonstante, Viskosität, Druckfestigkeit, pH-Wert, Leitfähigkeit, Kapazität, Dichte und/oder Temperatur.

Das Bereiten einer härtbaren Masse umfasst alle Formen und Arten der Bereitstellung der Masse entweder durch händisches, mechanisches oder automatisiertes Mischen unterschiedlicher Komponenten oder Ausbringen der Masse aus einem Behältnis und Initiierung des Härtevorgangs.

Unter In-Kontakt-Bringen ist das Berühren eines Teils der Oberfläche der härtbaren Masse mit der Oberfläche eines Substrats, auf das die Masse aufgebracht wurde, zumindest für die Dauer der Abbindereaktion zu verstehen.

Unter dem Begriff Anzeigeeinheit fallen alle Einheiten, die geeignet sind, den Benutzer der Vorrichtung über eine Zustandsänderung der Masse während des Härtens zu informieren, vorzugsweise in optischer und/oder akustischer Form. Umfasst werden Displays, insbesondere mit LED-Anzeigen, und Lautsprecher.

Eine Sensoreinheit im Sinne der Erfindung ist eine Einheit, die geeignet ist, irgendeine Zustandsänderung der Masse zu erfassen. Umfasst werden pH-Elektroden, Drehmomentaufnehmer, Schwingquarze, Thermoelemente, Widerstandsmesser, Kondensatoren, Dehnungsmessstreifen, Ultraschallsensoren. Die Abmessungen der Sensoreinheit sind grundsätzlich beliebig. Sensoren mit Baugrößen kleiner 5 mm sind bevorzugt.

Der Begriff Oberfläche umfasst alle Oberflächen, auf denen sich die härtbare Masse bestimmungsgemäß aufbringen lässt. Beispielsweise seien genannt: Abformlöffel, organisches Hartgewebe, wie Zahnsubstanz und Kiefer, dynamische Mischer. Nicht umfasst sind Oberflächen mit denen die Masse bei ihrer Herstellung oder vor Initiierung der eigentfichen Härtungsreaktion in Kontakt kommt.

Der Sensor befindet sich vorzugsweise in einer tragbaren Einheit. Vorzugsweise kann die Einheit, die den Sensor enthält, netzunabhängig betrieben werden.

Mit Kupplungselement sind Elemente gemeint, die es erlauben, die Vorrichtung zur Verfolgung des Abbindeverlaufs derart mit der härtbaren Masse in Beziehung zu setzen, dass ein reproduzierbares Ergebnis erhalten werden kann.

Insbesondere sind damit Elemente gemeint, die eine sichere Fixierung oder Befestigung der Vorrichtung in einem definierten Abstand zu einer Oberfläche oder fixiert in der auf die Oberfläche aufgebrachten Masse ermöglichen. Geeignete Kupplungselemente umfassen Gewinde, Clips, domförmige Fortsätze, Steckvorrichtungen, Magnete.

Die Erfindung weist dabei folgende Vorteile auf:

Das erfindungsgemäße Verfahren unter Verwendung der erfindungsgemäßen Vorrichtung ermöglicht das Verfolgen des Abbindevorgangs der Masse während des Härtens bei der bestimmungsgemäßen Anwendung "vor Ort", unabhängig davon, wann und wie die Masse gemischt wurde.

Die Erfindung eignet sich insbesondere im Dentalbereich zur Bestimmung des Endes der Verarbeitungszeit von dentalen Abformmassen.

Üblicherweise werden Dentalmassen durch Mischen einer Basis- und einer Katalysatorpaste bereitet. Abhängig vom Mischungsverhältnis und den Substanzen erfolgt der Abbindevorgang unterschiedlich schnell.

Das Mischen von Dentalmassen erfolgt üblicherweise in einem statischen oder dynamischen Mischer, beispielsweise gemäß der DE. 90 17 323 U1 oder der WO-98/43727 A1. Dieser wird entweder auf eine entsprechende Kartusche aufgesteckt und/oder mit einem elektrisch betriebenen Mischgerät, in das Kartuschen eingelegt werden können, betrieben. Geeignete Mischgeräte sind in der DE 29 906 343 U1 oder der EP 0 492 413 A1 beschrieben. Bei den zum Einsatz kommenden Mischern handelt es sich um Einmal-Mischer, da die gehärtete Masse aus dem Mischer nicht. mehr restlos ohne Zerstörung des Mischers entfemt werden kann.

Im Unterschied zu im industriellen Bereich zum Einsatz kommenden Messmischern oder Fördereinrichtungen mit Sensoren zur Überwachung der Einhaltung bestimmter rheologischer Eigenschaften der zu fördernden Masse zeichnet sich die Vorrichtung der vorliegenden Erfindung dadurch aus, dass die Bestimmung der Veränderung einer rheologischen Eigenschaft der härtbaren Masse entweder mit einer vom Mischer und vom Mischvorgang unabhängig betreibbaren Vorrichtung stattfindet oder der Bereich bzw. Abschnitt der zum Mischen verwendeten Vorrichtung, der mit der gemischten Masse in Kontakt kommt, als Wegwerf- bzw. Einwegartikel ausgebildet ist.

Mit einen durch Mischen erhaltenen dentalen Abformmasse, wird ein dentaler Abformlöffel befüllt, der anschließend in den Mundraum eines Patienten eingesetzt wird. Nach dem Abbinden wird der Abformlöffel aus dem Mund entnommen und von der Abformung ein Positivmodell angefertigt. Wird der Abformlöffel vor dem Ende der Verarbeitungszeit bzw. dem Ende der Abbindung entnommen, ist das Ergebnis unbrauchbar. Belässt man den Abformlöffel zu lange im Mund des Patienten, ist die Entnahme deutlich erschwert. Die Kenntnis über den günstigsten Zeitpunkt der Entnahme ist somit wichtig. Hinzu kommt, dass die physische und psychische Belastung des Patienten auf das notwendige Minimum herabgesetzt werden kann.

Insbesondere bei der Abformung von organischem Hartgewebe unter Verwendung von Abformmassen ist es wichtig, dass während des Abbindevorgangs bzw. während der Härtereaktion die Abformmasse im Bereich der abzuformenden Oberfläche nicht bewegt wird, um eine detailgetreue Abformung sicherstellen zu können.

Die Erfindung ermöglicht somit die Bestimmung des Endes der Verarbeitungszeit vorzugsweise unter den Bedingungen, die im Mundraum des Patienten herrschen mit der Folge, dass die erhärtete Masse einen optimalen Abdruck liefert, da sichergestellt werden kann, dass sie nicht vor dem Ende der Abbindung entnommen wird.

Das Ende der Verarbeitungszeit der Masse kann beispielsweise auf folgende Weise bestimmt werden:

Zur Bestimmung des Abbindeverlaufs wird die Vorrichtung zweckmäßigerweise an der Stelle mit der Oberfläche eines Abformlöffels verbunden, an der die härtende .Masse am kältesten ist. Dies stellt sicher, dass der Abbindeverlauf in dem Bereich der Masse verfolgt wird, die zuletzt erhärtet.

Die Vorrichtung ist ferner vorzugsweise chemisch und/oder thermisch sterilisierbar.

In einer bevorzugten Ausführungsform weist die Vorrichtung einen Sender auf, der die von der Sensoreinheit erfassten Daten drahtlos an eine Empfangseinheit übermittelt, die von der erfindungsgemäßen Vorrichtung getrennt ist. Dies ermöglicht eine weitere Miniaturisierung der Vorrichtung einerseits und andererseits eine externe Überwachung des Abbindeverlaufs. Die Übertragung der Daten kann aber auch auf herkömmliche Weise über ein Verbindungskabel erfolgen.

Der einen Abdruck von einer Zahnreihe anfertigende Zahnarzt kann auf diese Weise beispielsweise den Abbindeverlauf über ein tragbares Empfangsgerät aus einem anderen Sprechzimmer verfolgen: Die manuelle Überwachung durch Abtasten der Abformmasse im Mund des Patienten ist nicht mehr erforderlich.

Denkbar ist auch, die Spannungsversorgung, die Elektronik und den Sensor in miniaturisierter Bauweise in einen Abformlöffel, integrierbar zu gestalten. Die Datenübertragung kann dann beispielsweise über Telemetrie an das Mischgerät erfolgen. Im Mischgerät selbst befindet sich die Auswerteeinheit, die das Ende der Verarbeitungszeit der härtbaren Masse akustisch oder optisch anzeigt.

Gegebenenfalls verfügt die Vorrichtung selbst auch über eine Anzeigeeinheit, die auf optische und/oder akustische Weise den Anwender über den Abbindeverlauf informiert. Ausreichend kann sein, wenn die Anzeigeeinheit nur dann ein Signal abgibt, wenn ein voreinstellbarer Schwellenwert erreicht wird. Denkbar ist aber auch eine kontinuierliche information.

Üblicherweise weist die Vorrichtung auch einen Schalter oder Taster auf, mit dem der Messvorgang gestartet wird.

Als Sensoreinheit hat sich beispielsweise ein Schwingquarz, bewährt, der eine Torsions- und/oder Axialschwingung erzeugt, die durch die viskosen Eigenschaften der erhärtenden Masse gedämpft wird. Der Sensorkopf wird beispielsweise in Form eines Fühlers durch eine Öffnung an der Vorderseite eines Abformlöffels in die Masse eingeführt. Die Elektronik und Energieversorgung sind beispielsweise auswechselbar in den Abformlöffel integriert oder integrierbar.

Als Miniatursensor eignet sich auch ein Kondensator, wobei zwischen den Elektroden bzw. Kondertsatorplatten die härtbare Masse eingebracht wird. Der Elektrodenabstand ist hierbei konstant. Während des Härtens der Masse verändert sich die relative Dielektrizitätskonstante und damit die Kapazität des Kondensators. Der Kondensator kann beispielweise als Plattenkondensator ausgebildet sein. Denkbar sind aber auch Röhrenkondensatoren. Sensoren, die auf der Wirkungsweise eines idealisierten Plattenkondensators basieren sind bekannt (z. B. System capaNCDT der Fa. Micro-Epsilon).

Bevorzugte Ausführungsbeispiele werden nachstehend anhand der Zeichnungen erläutert.
- Figur 1: zeigt eine Ausführungsform, die zur Verfolgung des Abbindevorgangs als Sensor einen Plattenkondensator verwendet.
- Figur 2, 3: zeigen Ausführungsformen, die es erlauben, den Abbindevorgang berührungslos zu verfolgen.
- Figur 4: zeigt eine Messkurve, erhalten durch Auftragen des Ausgangssignals eines kapazitiven Sensors gegen die Verarbeitungszeit der härtbaren Masse.
- Figur 5: zeigt eine mögliche Ausführungsform für eine tragbare Messvorrichtung.
- Figur 6: zeigt eine Ausführungsform, in der die Vorrichtung von Figur 5 in einen dentalen Abformlöffel integriert ist.

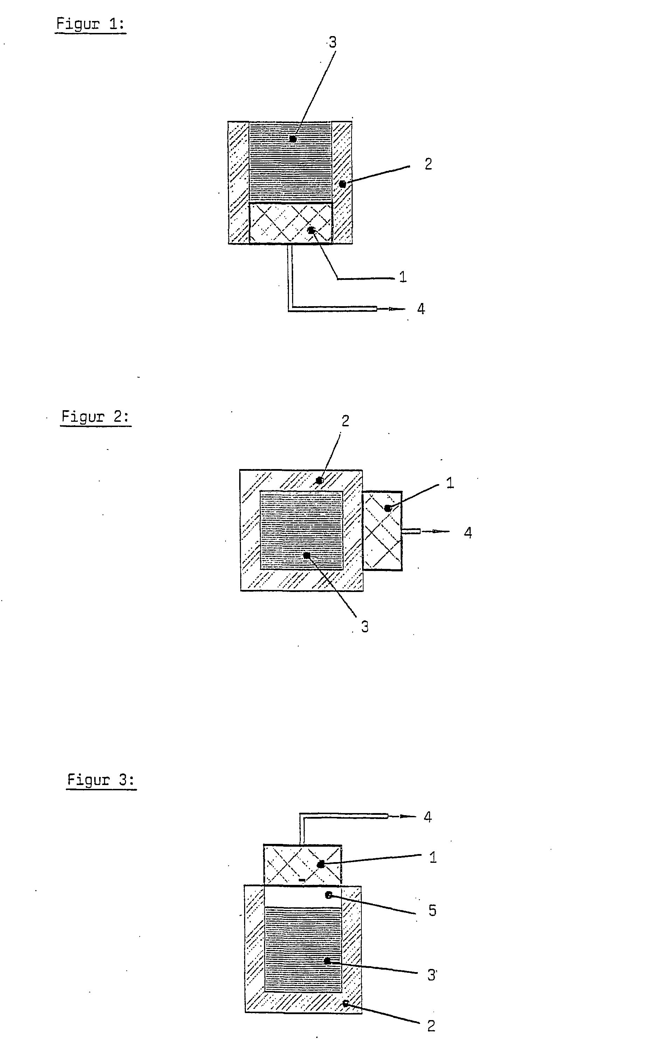

In der bevorzugten Ausführungsform gemäß Figur 1 wird über den Sensor (1) eine Hülse (2), beispielsweise aus Messing, Metall oder Kunststoff gesteckt, um ein definiertes Messvolumen zu erhalten. Dieses ist festgelegt-durch Sensorquerschnitt und Höhe der Hülse. Die so bereitgestellte Messkammer wird mit der Dentalmasse (3) bis zum Rand befüllt. Die zu untersuchende Masse besitzt somit eine definierte Schichtdicke ( = Höhe der Hülse). Die Hülse selbst hat keinen signifikanten Einfluss auf die Messung, solange sie nicht im Bereich der Fetdiinien liegt. Über eine Verbindung (4) steht die Sensoreinheit mit einer Elektronik in Verbindung.

In der Ausführungsform gemäß Figur 2 erfolgt die Verfolgung des Abbindevorgangs über den Sensor berührungslos. Der kapazitiven Sensor hat in dieser Ausführungsform keinen unmittelbaren Kontakt mit der härtbaren Dentalmasse. Die zu vermessende Dentalmasse (3) befindet sich in einer separaten Messkammer (2), die beispielsweise aus Kunststoff gefertigt ist. Der Sensor (1) erfasst in dieser Ausführungsform neben der zu vermessenden Masse selbst noch die Kapazität der Gehäusewandung der Messkammer (2). Da sich die Kapazität der Gehäusewandung während der Abbindung nicht ändert, wird die relative Veränderung der Ausgangsspannung nur durch Änderung der relativen Dielektrizitätskonstanten der erhärtenden Masse erzeugt. Das Verfahren kann somit als berührungslos betrachtet werden.

Denkbar ist auch, dass die Messkammer (2) offen und der kapazitive Sensor (1) nur durch eine Luftschicht (5) von der zu vermessenden Dentalmasse getrennt ist (Figur 3).

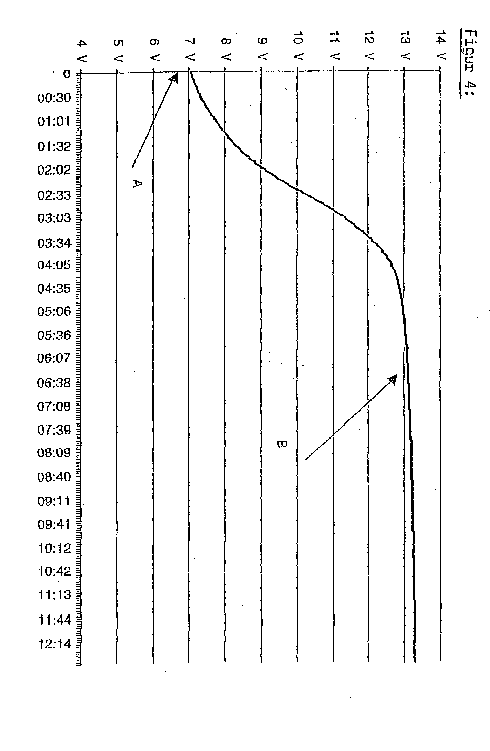

In Figur 4 ist das Ausgangssignal (Volt) des kapazitiven Sensors in Abhängigkeit von der Verarbeitungszeit (Minuten) aufgetragen. Zum Zeitpunkt A ist die Dentalmasse noch viskos, zum Zeitpunkt B ausgehärtet. Man erhält eine zunächst proportional ansteigende, Ausgangsspannung, die sich gegen Ende der Verarbeitungszeit der Masse asymptotisch einem Grenzwert nähert. Die Zeit, in der sich die Ausgangsspannung um einen definierten Wert relativ zum Ausgangswert ändert, korreliert mit der Verarbeitungszeit. Der Verlauf der Ausgangsspannung als Funktion der Verarbeitungszeit ermöglicht darüber hinaus auch Aussagen über die Abbindecharakteristik der vermessenen Masse (Steigung der Messkurve).

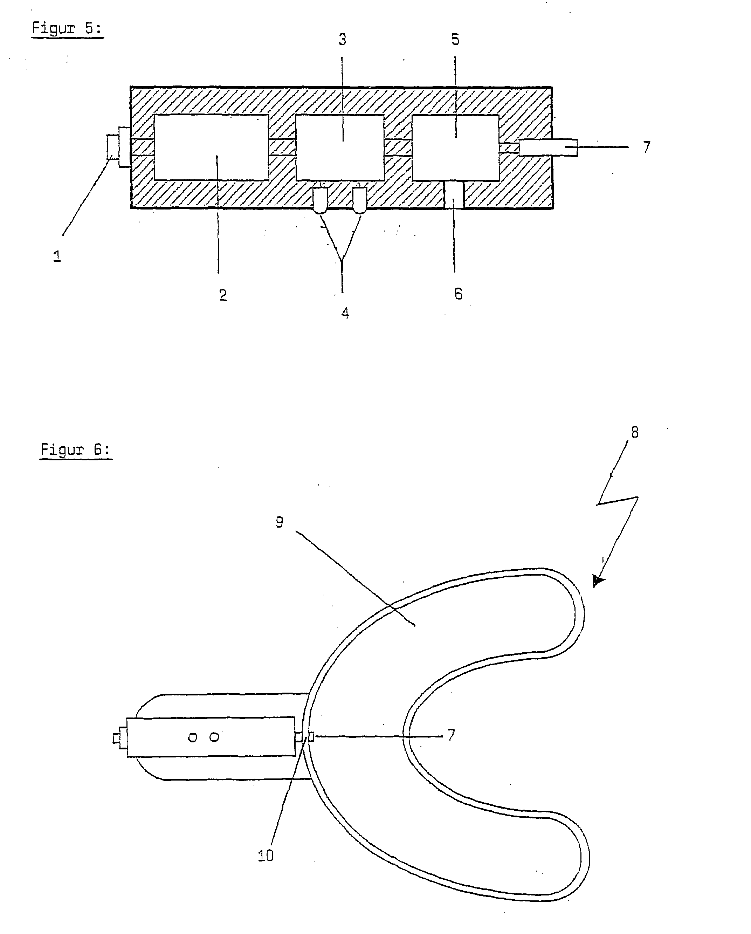

Eine Vorrichtung zur Bestimmung des Endes der Verarbeitungszeit von härtbaren Dentalmassen, die mit einer Oberfläche in Kontakt gebracht worden sind, weist gemäß Figur 5 beispielsweise einen Schalter (1), eine Spannungsversorgung (2), eine Auswerteelektronik (3), eine optische Anzeige (4), eine Ansteuerelsktronik (5), ein Trimmpotentiometer (6) und eine Sensoreinheit (7) auf, beispielsweise einen Schwingquarz oder einen kapazitiven Sensor.

In den Löffel gemäß Figur 6 ist die Vorrichtung nach Figur 5 integriert bzw. daran angebracht. Der Löffel (8) weist eine üblicherweise rinnenförmige Oberfläche (9) auf, auf die die härtbare Dentalmasse aufgetragen wird. Im vorderen Bereich des Löffels befindet sich eine Öffnung (10), über die der Schwingquarz (7) in die Masse eintauchen kann. Die Vorrichtung ist beispielsweise über einen Magneten, eine Clip oder ein Gewinde an den Löffel ankoppelbar.

## Patentansprüche

1. Vorrichtung zur Verfolgung des Abbindevorgangs bzw. zur Bestimmung des Endes der Verarbeitungszeit von härtbaren Dentalmassen, die in einen dentalen Abformlöffel ausgebracht wurden, umfassend eine Spannungsversorgung, eine Sensoreinheit und ein Kupplungselement, wobei die Sensoreinheit mindestens eine Veränderung mindestens einer Eigenschaft der härtbaren Dentalmasse während des Härtens erfasst, wobei die Vorrichtung über ein Kupplungselement mit einem Abformlöffel verbunden ist.

2. Vorrichtung nach Anspruch 1, wobei die Sensoreinheit zur Bestimmung geeignet ist von: Dielektrizitätskonstante, Viskosität, Druckfestigkeit, pH-Wert, Leitfähigkeit, Kapazität, Dichte, Temperatur und/oder Impedanz.

3. Vorrichtung nach Anspruch 1 oder 2, wobei das Kupplungselement die Form eines Clips, eines Gewindes, einer Steckvorrichtung, eines domförmigen Fortsatzes aufweist oder magnetisch ist, und das Anbringen der Vorrichtung in definierter Weise ermöglicht..

4. Vorrichtung nach einem der vorstehenden Ansprüche mit einer Übertragungseinheit, die die von der Sensoreinheit erfassten Werte an einen Empfänger übermittelt.

5. Vorrichtung nach einem der vorstehenden Ansprüche, wobei die Sensoreinheit gewählt ist aus: Schwingquarz, Kondensator, Thermometer, pH-Elektrode, Drehmomentaufnehmer, Thermoelement, Widerstandsmesser, Dehnungsmessstreifen, Ultraschalisensoren.

6. Vorrichtung nach einem der vorstehenden Ansprüche, wobei der Sensor netzunabhängig betrieben werden kann.

7. Vorrichtung nach einem der vorstehenden Ansprüche, wobei der Abformlöffel eine Öffnung, aufweist, über die die Sensoreinheit der Vorrichtung eingeführt werden kann.

8. Verwendung einer Vorrichtung nach einer der vorstehenden Ansprüche zur Verfolgung des Abbindeverlaufs von Dentalmassen.

9. Verfahren zur Verfolgung des Abbindevorgangs bzw. zur Bestimmung des Endes der Verarbeitungszeit von härtbaren Dentalmassen umfassend die Schritte: a) Bereitstellen der Vorrichtung und eines Abformlöffels, b) Befüllen des Abformlöffels mit einer härtbaren Masse, c) Anfertigung einer Abformung, wobei vor oder während einer der Schritte b) oder c) die Vorrichtung gemäß einer der Ansprüche 1 bis 6 mit dem Abformlöffel gekoppelt wird.

10. Verfahren nach Anspruch 9, umfassend Schritt d) Abgabe eines Signals sobald ein einstellbarer Schwellenwert überschritten wird.

11. Verfahren nach einem der Ansprüche 9 bis 10, wobei das Ende der Verarbeitungszeit nach initiierung einer Härtungsreaktion im Bereich von 0,1 bis 60 min erreicht ist.

12. Verfahren nach einem der Ansprüche 9 bis 11, wobei Schritt c) in einem Temperaturbereich von 15 bis 50°C durchgefürt wird.

13. Verfahren nach Anspruch 9, **dadurch gekennzeichnet, daß** Schritt c) Erfassen mindestens einer Anderung mindestens einer Eigenschaft der härtbaren Dentalmasse, die mit der Oberfläche in Kontakt gebracht wurde, während des Härtens, d) Abgabe eines Signals sobald ein einstellbarer Schwellenwert überschritten wird, umfaßt.

14. Verfahren nach Anspruch 13, wobei die Oberfläche gewählt ist aus: Oberfläche eines Abformlöffel und von organischem Hartgewebe.

## Claims

1. Device for following the setting process or for determining the end of the processing time of hardenable dental compounds which have been dispensed into a dental impression tray, comprising a voltage supply, a sensor unit and a coupling element, the sensor unit sensing at least a change in at least one property of the hardenable dental compound during the hardening, the device being connected to an impression tray by means of a coupling element.

2. Device according to Claim 1, the sensor unit being suitable for the determination of: dielectric constant, viscosity, compressive strength, pH, conductivity, capacitance, density, temperature and/or impedance.

3. Device according to Claim 1 or 2, the coupling element having the form of a clip, a thread, a plug-in device, a spike-shaped continuation or being magnetic, and making it possible for the device to be attached in a defined way.

4. Device according to one of the preceding claims with a transmission unit, which transmits the values sensed by the sensor unit to a receiver.

5. Device according to one of the preceding claims, the sensor unit being chosen from: oscillator crystal, capacitor, thermometer, pH electrode, torque transducer, thermocouple, resistance meter, wire strain gage, ultrasonic sensors.

6. Device according to one of the preceding claims, it being possible for the sensor to be operated independently of the power supply system.

7. Device according to one of the preceding claims, the impression tray having an opening via which the sensor unit of the device can be introduced.

8. Use of a device according to one of the preceding claims for following the progress of the setting process of dental compounds.

9. Method for following the setting process or for determining the end of the processing time of hardenable dental compounds, comprising the steps: a) provision of the device and an impression tray, b) filling the impression tray with a hardenable compound, c) preparation of an impression, the device according to one of Claims 1 to 6 being coupled to the impression tray before or during one of the steps b) or c).

10. Method according to Claim 9, comprising step d) emission of a signal as soon as a settable threshold value is exceeded.

11. Method according to one of Claims 9 to 10, the end of the processing time being reached after initiation of a hardening reaction in the range from 0.1 to 60 minutes.

12. Method according to one of Claims 9 to 11, step c) being carried out in a temperature range from 15 to 50°C.

13. Method according to Claim 9, **characterized in that** step c) comprises sensing at least one change in at least one property of the hardenable dental compound which has been brought into contact with the surface during the hardening, d) comprises emission of a signal as soon as a settable threshold value is exceeded.

14. Method according to Claim 13, the surface being chosen from: surface of an impression tray and of hard organic tissue.

## Revendications

1. Dispositif pour suivre le processus de prise ou pour déterminer la fin du temps de traitement des masses dentaires durcissables qui ont été appliquées dans une cuiller de moulage dentaire, comprenant une alimentation électrique, une unité de détection et un élément de couplage, l'unité de détection détectant au moins une modification d'au moins une propriété de la masse dentaire durcissable pendant le durcissement, le dispositif étant relié à une cuiller de moulage par le biais d'un élément de couplage.

2. Dispositif selon la revendication 1, l'unité de détection convenant pour la détermination de : la constante diélectrique, la viscosité, la résistance à la compression, la valeur pH, la conductivité, la capacité, la densité, la température et/ou l'impédance.

3. Dispositif selon la revendication 1 ou 2, l'élément de couplage présentant la forme d'un clip, d'une filet, d'un dispositif enfichable, d'un prolongement en forme de goujon ou étant magnétique et permettant le montage du dispositif d'une manière donnée.

4. Dispositif selon l'une des revendications précédentes comprenant une unité de transmission qui transmet les valeurs détectées par l'unité de détection à un récepteur.

5. Dispositif selon l'une des revendications précédentes, l'unité de détection étant choisie parmi : un quartz d'oscillation, un condensateur, un thermomètre, une électrode de pH, un capteur dynamométrique, un élément thermostatique, un mesureur de résistance, une jauge de contrainte, des capteurs à ultrasons.

6. Dispositif selon l'une des revendications précédentes, le capteur pouvant fonctionner indépendamment du réseau.

7. Dispositif selon l'une des revendications précédentes, la cuiller de moulage présentant une ouverture par le biais de laquelle peut être introduite l'unité de détection du dispositif.

8. Application d'un dispositif selon l'une des revendications précédentes pour suivre l'évolution de la prise de masses dentaires.

9. Procédé pour suivre le processus de prise ou pour déterminer la fin du temps de traitement des masses dentaires durcissables comprenant les étapes suivantes : a) Fourniture du dispositif et d'une cuiller de moulage ; b) Remplissage de la cuiller de moulage avec une masse durcissable ; c) Réalisation d'un moulage, le dispositif conforme à l'une des revendications 1 à 6 étant couplé avec la cuiller de moulage avant ou pendant l'une des étapes b) ou c).

10. Procédé selon la revendication 9, comprenant l'étape d) Émission d'un signal dès qu'une valeur de seuil pouvant être préréglée est dépassée.

11. Procédé selon l'une des revendications 9 à 10, la fin du temps de traitement étant atteinte entre 0,1 et 60 minutes après avoir initié la réaction de durcissement.

12. Procédé selon l'une des revendications 9 à 11, l'étape c) étant effectuée dans une plage de température de 15 à 50 °C.

13. Procédé selon la revendication 9, **caractérisé en ce que** l'étape c) Détection, pendant le durcissement, d'au moins une modification d'au moins une propriété de la masse durcissable qui est mise en contact avec la surface, comprend d) Émission d'un signal dès qu'une valeur de seuil pouvant être préréglée est dépassée.

14. Procédé selon la revendication 13, la surface étant choisie parmi : la surface d'une cuiller de moulage et des tissus organiques durs.
